# EUROPÄISCHE PATENTANMELDUNG

(11) **EP 2 457 557 A2**
(43) Veröffentlichungstag der Anmeldung: **30.05.2012**
(21) Anmeldenummer: 11177914.6
(22) Anmeldetag: 18.08.2011
(51) Int. Cl.: A61K 8/35, A61K 8/37, A61K 8/40, A61K 8/49, A61K 8/55, A61K 8/92, A61Q 17/04, A61Q 19/08

(54) **Stabilisierte Emulsion**

(30) Priorität: 08.09.2010 DE 102010044681
(71) Anmelder: Beiersdorf AG, 20253 Hamburg (DE)
(72) Erfinder: Rathsack, Jessica, 21614 Buxtehude (DE); Neuhoff, Henrike, 22359 Hamburg (DE); Möllgaard, Svenja Lena, 22301 Hamburg (DE); Heike, Kerstin, 22457 Hamburg (DE)

(57) **Zusammenfassung**

Kosmetische Emulsion enthaltend
a) Kaliumcetylphosphat,
b) hydrierte Palmölglyceride,
c) 4-(tert.-Butyl)-4'-methoxydibenzoylmethan,
d) 2-Ethylhexyl-2-cyano-3,3-diphenylacrylat,
e) 2-Phenylbenzimidazol-5-sulfonsäure und/oder deren Salze.

## Beschreibung

Die vorliegende Erfindung betrifft eine kosmetische Emulsion enthaltend Kaliumcetylphosphat, hydrierte Palmölglyceride, 4-(tert.-Butyl)-4'-methoxydibenzoylmethan, 2-Ethylhexyl-2-cyano-3,3-diphenylacrylat, 2-Phenylbenzimidazol-5-sulfonsäure und/oder deren Salze.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte bestehen in der Regel aus Emulsionen. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion. Der Grundcharakter einer O/W-Emulsion ist durch das Wasser geprägt. Bei einer Wasser-in-Öl-Emulsion handelt es sich um das umgekehrte Prinzip, wobei der Grundcharakter hier durch das Öl bestimmt wird.

Ein klassischer Emulgator für die Herstellung von Emulsionen ist Kaliumcetylphosphat in Kombination mit hydrierten Palmölglyceriden.

Um kosmetischen Zubereitungen einen UV-Schutz zu verleihen, werden diesen Zubereitungen UV-A-und/oder UV-B-Filter zugesetzt. Bekannte UV-Filter sind beispielsweise 4-(tert.-Butyl)-4'-methoxydibenzoylmethan und 2-Ethylhexyl-2-cyano-3,3-diphenylacrylat (INCI Octocrylen).

Nachteilig am Stande der Technik ist jedoch der Umstand, dass kosmetische Emulsionen, insbesondere O/W-Emulsionen häufig sehr dünnflüssig sind. Will man diese Zubereitungen dann beispielsweise aus einem Vorratsbehältnis entnehmen und im Gesicht auftragen, rinnen sie einem "durch die Finger" und tropfen unkontrolliert Richtung Erdboden.

Um derartige Probleme im wahrsten Sinne des Wortes in den "in den Griff" zu bekommen , werden diesen Emulsionen Konsistenzbildner (Verdickungsmittel) zugesetzt, beispielsweise Polyacrylate, Carrageenan und ähnliches, welche der Zubereitung eine entsprechende Viskosität und Fließgrenze geben.

Derartige Verdickungsmittel sind jedoch relativ teuer, verursachen Probleme bei der Herstellung der Zubereitung und können, wie letztendendes jede Substanz zu Nebenreaktionen mit anderen Bestandteilen der Zubereitung oder der Haut führen.

Insbesondere bei der Herstellung der Emulsion sind zusätzliche zeit-und energieaufwendige Zusatzschritte notwendig, wenn ein Verdickungsmittel eigearbeitet werden soll. Häufig müssen diese Verbindungen "vorgequollen" werden. Nach der Einarbeitung darf nur noch mit reduzierten Scherkräften gerührt/homogenisiert werden, damit das Gelgerüst keinen irreparablen Schaden nimmt. Dadurch verlängert sich zeitlich der Herstellungsprozess.

Es war daher die Aufgabe der vorliegenden Erfindung, die Mängel des Standes der Technik zu beseitigen oder zumindest zu mindern und eine Emulsion auf der Basis von Kaliumcetylphosphat/hydrierten Palmölglyceriden zu entwickeln, die eine höhere Viskosität aufzeigt als die Zubereitungen des Standes der Technik.

Überraschend gelöst wird die Aufgabe durch eine kosmetische Emulsion enthaltend
a) Kaliumcetylphosphat,
b) hydrierte Palmölglyceride,
c) 4-(tert.-Butyl)-4'-methoxydibenzoylmethan,
d) 2-Ethylhexyl-2-cyano-3,3-diphenylacrylat,
e) 2-Phenylbenzimidazol-5-sulfonsäure und/oder deren Salze.

Überraschend gelöst wird die Aufgabe ferner durch die Verwendung von 2-Phenylbenzimidazol-5-sulfonsäure und/oder deren Salzen zur Erhöhung der Viskosität von Emulsionen auf der Basis von Kaliumphosphat und hydrierten Palmölglyceriden.

Als Salze werden erfindungsgemäß üblicherweise Natrium-, Kalium-, Trialkylammonium- und/oder Triethanolammoniumsalze eingesetzt.

Zwar kennt der Stand der Technik u.a. die DE 102006055042, DE 102006056320, EP 0514491, EP 1280505, EP 0780119 und US 5738841, doch konnten diese Schriften nicht den Weg zur vorliegenden Erfindung weisen.

Als "erfindungsgemäß", "erfindungsgemäß vorteilhaft" etc. gekennzeichnete Ausführungsformen beziehen sich im Rahmen dieser Offenbarung sowohl auf die erfindungsgemäße Zubereitung als auch auf die erfindungsgemäße Zusammensetzung.

Als erfindungsgemäße hydrierte Palmölglyceride werden erfindungsgemäß bevorzugt Palmölglyceride mit der CAS Nr. 91744-66-0; Einecs: 294-631-0 eingesetzt, die beispielsweise von der Firma Symrise unter dem Handelsnamen Emulsiphos angeboten werden.

Erfindungsgemäß vorteilhafte Ausführungsformen der vorliegenden Erfindung sind dadurch gekennzeichnet, dass die Zubereitung Kaliumcetylphosphat in einer Konzentration von 0.1 bis 10 Gew.-%, bezogen auf das Gesamtgewicht der Emulsion, enthält.

Erfindungsgemäß bevorzugte Ausführungsformen der vorliegenden Erfindung sind dadurch gekennzeichnet, dass die Zubereitung Kaliumcetylphosphat in einer Konzentration von 0.25 bis 4 Gew.-%, bezogen auf das Gesamtgewicht der Emulsion, enthält.

Es ist erfindungsgemäß vorteilhaft, wenn die Zubereitung hydrierte Palmölglyceride in einer Gesamtkonzentration von 0.1 bis 10 Gew.-%, bezogen auf das Gesamtgewicht der Emulsion, enthält.

Es ist erfindungsgemäß bevorzugt, wenn die Zubereitung hydrierte Palmölglyceride in einer Gesamtkonzentration von 0.25 bis 4 Gew.-%, bezogen auf das Gesamtgewicht der Emulsion, enthält.

Es ist vorteilhaft im Sinne der vorliegenden Erfindung, wenn die Zubereitung 2-Ethylhexyl-2-cyano-3,3-di-phenylacrylat in einer Gesamtkonzentration von 0.05 bis 10 Gew.-%, bezogen auf das Gesamtgewicht der Emulsion, enthält.

Es ist bevorzugt im Sinne der vorliegenden Erfindung, wenn die Zubereitung 2-Ethylhexyl-2-cyano-3,3-di-phenylacrylat in einer Gesamtkonzentration von 0.5 bis 5 Gew.- %, bezogen auf das Gesamtgewicht der Emulsion, enthält.

Erfindungsgemäß vorteilhafte Ausführungsformen der vorliegenden Erfindung sind dadurch gekennzeichnet, dass die Zubereitung 4-(tert.-Butyl)-4'-methoxydibenzoylmethan in einer Gesamtkonzentration von 0.01 bis 5 Gew.-%, bezogen auf das Gesamtgewicht der Emulsion, enthält.

Erfindungsgemäß bevorzugte Ausführungsformen der vorliegenden Erfindung sind dadurch gekennzeichnet, dass die Zubereitung 4-(tert.-Butyl)-4'-methoxydibenzoylmethan in einer Gesamtkonzentration von 0.5 bis 4 Gew.-%, bezogen auf das Gesamtgewicht der Emulsion, enthält.

Es ist erfindungsgemäß vorteilhaft, wenn die Zubereitung 2-Phenylbenzimidazol-5-sulfonsäure und/oder deren Salze in einer Gesamtkonzentration von 0,1 bis 10 Gew.-%, bezogen auf das Gesamtgewicht der Emulsion, enthält.

Es ist erfindungsgemäß bevorzugt, wenn die Zubereitung 2-Phenylbenzimidazol-5-sulfonsäure und/oder deren Salze in einer Gesamtkonzentration von 0.25 bis 5 Gew.-%, bezogen auf das Gesamtgewicht der Emulsion, enthält.

Es ist vorteilhaft im Sinne der vorliegenden Erfindung, wenn die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin (INCI :Bis-Ethylhexyloxyphenol Methoxyphenyl Triazin); Dioctylbutylamidotriazon (INCI: Diethylhexyl-Butamidotriazone); 2,4-bis-[5-1 (dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyitriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; hydrophobes Titiandioxid; hydrophobes Zinkoxid in einer Konzentration von 0,01 bis 40 Gewichts-% bezogen auf das Gesamtgewicht der Zubereitung. Erfindungsgemäß vorteilhafte Ausführungsformen der vorliegenden Erfindung sind auch dadurch gekennzeichnet, dass die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, ß-Alanin, Tocopherylacetat, Harnstoff; Hyaluronsäure; Dihydroxyaceton; 8-Hexadecen-1, 16-dicarbonsäure und/oder Licochalcon A enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9), Talkum, Lauroyl Lysine und Acrylonitrilemethacrylonitrile-methyl-methacrylate.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, 2-Methylpropan-1,3-diol, Glycerin, Ethylenglykol, Ethylenglykolmonoethyl- oder-monobutylether, Propylenglykolmonomethyl, -monoethyl- oder -monobutylether, Diethylenglykolmonomethyl- oder -monoethylether und analoge Produkte, Schaumstabilisatoren, Elektrolyte, etc..

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung Cetiol CC bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol

Dicaprylat/Dicaprat, C₁₂₋₁₃-Alkyllactat, Di-C₁₂₋₁₃-Alkyltartrat, Trüsostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Vorteilhaft im Sinne der vorliegenden Erfindung sind Zubereitungen zur Pflege der Haut. Sie können dem kosmetischen Lichtschutz, der Hautpflege (beispielsweise zur Prophylaxe und Behandlung der Hautalterung) und dekorativen Kosmetik dienen. Bevorzugt ist der Einsatz in Tagespflegeprodukten.

### Vergleichsversuche

Mit den folgenden Vergleichsversuchen konnte der erfindungsgemäße Effekt belegt werden:
Es wurde eine O/W-Emulsion mit 1,5 Gew.-% Kaliumcetylphosphat, 1 Gew.-% 4-(tert.-Butyl)-4'-methoxydibenzoylmethan, 1 Gew.-% 2-Ethylhexyl-2-cyano-3,3-diphenylacrylat sowie 1 Gew.-% hydrierte Palmölglyceriden hergestellt und mit der angegebenen Menge (in Gew.-%) an neutralisierter 2-Phenylbenzimidazol-5-sulfonsäure versetzt. Anschließend wurde die Viskosität mittels eines Konsistenzmessgerätes nach Figge (welches in der DE 2909087 offenbart ist) bestimmt. Dabei bedeutet eine höhere Zahl von Skalenteilen eine höhere Viskosität.

| Konzentration Phenylbenzimidazol-5-sulfonsäure | 1 Gew.-% | 2 Gew.-% |
|---|---|---|
| Viskosität nach 1 Tag in Skalenteilen | 9 | 12 |

### Beispiele

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

| INCI | Beispiel 1 | Beispiel 2 | Beispiel 3 | Beispiel 4 |
|---|---|---|---|---|
| Cyclomethicone | 1 | 2 | 2,5 | 3 |
| Ethylhexylglycerin | 0,2 | 0,5 | 0,3 | 0,4 |
| Tocopheryl Acetate | 1 | 1 | 2 | 0,5 |
| Propylene Glycol + Nelumbium Speciosum Flower Extract | 0,5 | 2 | 1 | 1 |
| Panthenol | 1 | 5 | 3 | 0,5 |
| Cetyl Alcohol | 0,25 | 1,5 | 3 | 2 |
| Dicaprylyl Ether | 2 | 3 | 4 | 5 |
| Butyrospermum Parkii Butter | 1 | 5 | 3 | 2 |
| Dimethicone | 0,5 | 1 | 2 | 0,5 |
| Cera Microcristallina + Paraffinum Liquidum | 2 | 1,5 | 4 | 3 |
| Cetearyl Alcohol | 2 | 3 | 4 | 5 |
| C12-15 Alkyl Benzoate | | 1 | 2 | |
| Glycine Soja Oil + Calendula Officinalis Flower Extract | 1 | 0,1 | 0,5 | 2 |
| Prunus Amygdalus Dulcis Oil | 1 | 2 | 0,5 | 3 |
| Caprylic/Capric Triglyceride | 1 | 2 | 1 | 3 |
| Potassium Cetyl Phosphate + Hydrogenated Palm Glycerides | 2,5 | 1 | 0,5 | 3 |
| Glyceryl Stearate | 2 | 2,5 | 3 | 4 |
| Tapioca Starch + Aqua | 1 | 2 | 3 | 0,5 |
| Nylon-12 | 1,5 | 3 | 4 | 1 |
| Parfum | 0,3 | 0,4 | 0,7 | 1 |
| Glycerin | 3 | 5 | 7 | 9 |
| Glyceryl Glucoside | 6 | 10 | 5 | 2,5 |
| Sodium Hydroxide | 1 | 1,5 | 1,5 | 1 |
| Propylparaben | 0,1 | 0,2 | 0,25 | 3 |
| Methylparaben | 0,2 | 0,25 | 3 | 0,2 |
| Phenoxyethanol | 0,6 | 0,4 | 0,4 | 0,5 |
| Ethylparaben | 0,2 | 0,1 | 0 | 0,3 |
| Methylpropanediol | 4 | 2 | 3 | 1 |
| Carbomer | 0,5 | 1 | 1,5 | 0,75 |
| Acrylates/C10-30 Alkyl Acrylate Crosspolymer | 0,4 | 0,2 | 0,5 | 0,3 |
| Chondrus Crispus | 0,3 | 0,4 | 0,2 | 0,1 |
| Acrylates/C10-30 Alkyl Acrylate Crosspolymer | 0,5 | 0,4 | 0,2 | 0,1 |
| Aqua | ad 100% | ad 100% | ad 100% | ad 100% |
| Alcohol Denat. | 2 | 1 | 3 | 4 |
| Trisodium EDTA | 0,5 | 1 | 2 | 1,5 |
| Octocrylene | 2 | 3 | 4 | 5 |
| Ethylhexyl Salicylate | 4 | 2 | 3 | 1 |
| Butyl Methoxydibenzoylmethane | 2 | 3 | 1 | 1,5 |
| Phenylbenzimidazole Sulfonic Acid | 2 | 3 | 4 | 1 |

## Patentansprüche

1. Kosmetische Emulsion enthaltend
a) Kaliumcetylphosphat,
b) hydrierte Palmölglyceride,
c) 4-(tert.-Butyl)-4'-methoxydibenzoylmethan,
d) 2-Ethylhexyl-2-cyano-3,3-diphenylacrylat,
e) 2-Phenylbenzimidazol-5-sulfonsäure und/oder deren Salze.

2. Verwendung von 2-Phenylbenzimidazol-5-sulfonsäure und/oder deren Salzen zur Erhöhung der Viskosität von Emulsionen auf der Basis von Kaliumphosphat und hydrierten Palmölglyceriden.

3. Kosmetische Emulsion nach Anspruch 1 oder Verwendung nach Anspruch 2, **dadurch gekennzeichnet, dass** die Zubereitung Kaliumcetylphosphat in einer Konzentration von 0,1 bis 10 Gew.-%, bezogen auf das Gesamtgewicht der Emulsion, enthält.

4. Kosmetische Emulsion oder Verwendung nach einem der vorhergehenden Ansprüche, **dadurch gekennzeichnet, dass** die Zubereitung hydrierte Palmölglyceride in einer Gesamtkonzentration von 0,1 bis 10 Gew.-%, bezogen auf das Gesamtgewicht der Emulsion, enthält.

5. Kosmetische Emulsion oder Verwendung nach einem der vorhergehenden Ansprüche, **dadurch gekennzeichnet, dass** die Zubereitung 2-Ethylhexyl-2-cyano-3,3-diphenylacrylat in einer Gesamtkonzentration von 0,05 bis 10 Gew.-%, bezogen auf das Gesamtgewicht der Emulsion, enthält.

6. Kosmetische Emulsion oder Verwendung nach einem der vorhergehenden Ansprüche, **dadurch gekennzeichnet, dass** die Zubereitung 4-(tert.-Butyl)-4'-methoxydibenzoylmethan in einer Gesamtkonzentration von 0,01 bis 5 Gew.-%, bezogen auf das Gesamtgewicht der Emulsion, enthält.

7. Kosmetische Emulsion oder Verwendung nach einem der vorhergehenden Ansprüche, **dadurch gekennzeichnet, dass** die Zubereitung 2-Phenylbenzimidazol-5-sulfonsäure und/oder deren Salze in einer Gesamtkonzentration von 0,1 bis 10 Gew.-%, bezogen auf das Gesamtgewicht der Emulsion, enthält.

8. Kosmetische Emulsion oder Verwendung nach einem der vorhergehenden Ansprüche, **dadurch gekennzeichnet, dass** die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2- bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin (INCI :Bis-Ethylhexyloxyphenol Methoxyphenyl Triazin); Dioctylbutylamidotriazon (INCI: Diethylhexyl-Butamidotriazone); 2,4-bis-[5-1 (dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; hydrophobes Titiandioxid; hydrophobes Zinkoxid in einer Konzentration von 0,01 bis 40 Gewichts-% bezogen auf das Gesamtgewicht der Zubereitung.

9. Kosmetische Emulsion oder Verwendung nach einem der vorhergehenden Ansprüche, **dadurch gekennzeichnet, dass** die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, ß-Alanin, Tocopherylacetat, Harnstoff; Hyaluronsäure; Dihydroxyaceton; 8-Hexadecen-1,16-dicarbonsäure und/oder Licochalcon A enthält.
